# EUROPEAN PATENT APPLICATION

(11) **EP 4 467 532 A1**
(43) Date of publication of application: **27.11.2024**
(21) Application number: 23773457.9
(22) Date of filing: 31.01.2023
(51) Int. Cl.: C07D 221/14, C07D 401/12, A61K 31/496, A61P 35/00

(54) **DIIMIDE DERIVATIVE, PREPARATION METHOD THEREFOR AND USE THEREOF**

(30) Priority: 23.03.2022 CN 202210298748
(71) Applicant: Xiangbei Welman Pharmaceutical Co., Ltd, Hunan 410331 (CN); Guangzhou Century Clinical Research Co., Ltd, Guangzhou, Guangdong 510535 (CN); Guangzhou Xin-Chuangyi Biopharmaceutical Co., Ltd, Huangpu District Guangzhou, Guangdong 510535 (CN); Guangzhou Xin Chuang Yi New Drug R&D Co., Ltd, Guangzhou, Guangdong 510535 (CN); Nanjing Kangfushun Pharmaceutical Co., Ltd, Nanjing, Jiangsu 210046 (CN)
(72) Inventor: SUN, Tianyu, Guangzhou, Guangdong 510620 (CN); CHU, Jiegen, Guangzhou, Guangdong 510620 (CN)
(74) Representative: Kraus & Lederer PartGmbB
(86) International application number: PCT/CN2023/074050
(87) International publication number: WO 2023/179205

(57) **Abstract**

Provided are a diimide derivative, a preparation method therefor and the use thereof. The diimide derivative has a good anti-tumor activity, and the anti-proliferative activity against various cancer cells is significantly better than those of similar compounds. (I)

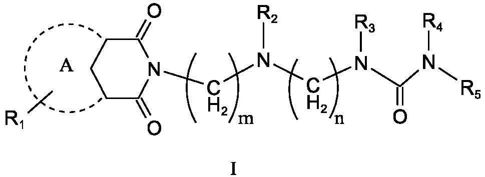

## Description

### TECHNICAL FIELD

The present disclosure relates to a diimide derivative, and a preparation method therefor and use thereof, belonging to the field of medicinal chemistry.

### BACKGROUND

Diimide compounds are an important class of substances in the field of medicinal chemistry and have many interesting pharmacological activities.

Further fusion of diimide with some fused rings can form a unique planar structure with a greatly reduced molecular volume which can be embedded between base pairs of deoxyribonucleic acid (DNA) double strands, and then causing the unwinding of the DNA double helix, thus having a unique application value for some cell proliferative diseases, such as cancers. The most common fused rings fused to the diimide are naphthalene and anthracene. Many compounds having these structures have reported antitumor activity, but fewer compounds have entered the clinical stage.

Amonafide is one of the representative compounds and is reported to have strong cytotoxic activity against various cancer cells such as colon cancer, lung cancer, gastric cancer, esophageal cancer and leukemia. Antisoma Company has advanced amonafide into Phase III clinical trials for the treatment of acute myeloid leukemia, but the development for amonafide is ultimately terminated due to its poor efficacy.

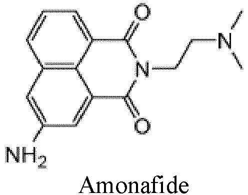

Currently, other diimide compounds are still being researched in the industry in order to obtain better potential compounds, but it is still difficult to find a compound with a stronger activity than that of amonafide.

### SUMMARY

An objective of the present disclosure is to provide a fused ring diimide compound which has better properties.

In a first aspect, the present disclosure provides a compound having a diimide structure represented by formula I, 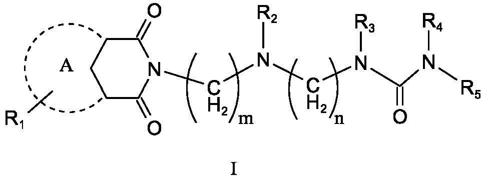
wherein in formula I,
A is naphthalene or anthracene, fused with diimide through 3 carbon atoms;
R₁ is hydrogen, alkyloxy or nitro;
m or n is 1, 2 or 3, and m+n ≤ 4;
R₂ is alkyl;
R₃ is hydrogen or alkyl; and
R₄ or R₅ is alkyl, haloalkyl or nitroso.

In addition, in formula I, R₃ optionally forms a ring together with R₂.

In some embodiments, in the formula I, the alkyl is C1-C4 alkyl.

In some embodiments, in the formula I, the alkyloxy is C1-C4 alkyloxy.

In some embodiments, in the formula I, the haloalkyl group is C1-C4 haloalkyl.

In some embodiments, in the formula I, A is preferably naphthalene, and some preferred compounds of formula I have superior properties, such as stronger activity.

In some embodiments, in the formula I, R₁ is preferably nitro or C1-C4 alkyloxy, and more preferably nitro. Some preferred compounds of formula I have superior properties, such as stronger activity.

In some embodiments, in the formula I, R₃ is preferably hydrogen, and some preferred compounds of formula I have superior properties, such as stronger activity.

In some embodiments, in the formula I, R₂ forms a ring (a five-membered ring, a six-membered ring, *etc*.) together with R₃. For example, R₃ and R₂ together with the nitrogen atom to which they are attached form a saturated nitrogen heterocycle.

In some embodiments, in the formula I, R₄ is preferably C1-C4 haloalkyl, and R₅ is preferably nitroso. Some preferred compounds of formula I have superior properties, such as stronger activity.

In some embodiments, in the formula I, m and n are preferably: m=1 and n=3, m=1 and n=2, or m=2 and n=2, more preferably, m=2 and n=2. Some preferred compounds of formula I have superior properties, such as stronger activity.

In some embodiments, in the formula I, the C1-C4 alkyl is selected from the group consisting of methyl, ethyl, n-propyl, isopropyl, n-butyl, sec-butyl, isobutyl, tert-butyl, etc. Halogen is selected from the group consisting of fluorine, chlorine, and bromine.

In a second aspect, the present disclosure provides a method for preparing the compound of formula I, comprising: 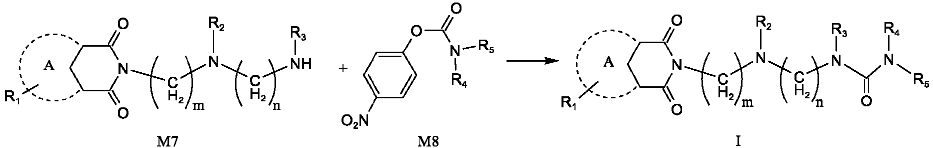 reacting a compound of formula M7 with a compound of formula M8 in the presence of a base to generate a compound of formula I according to the above reaction formula, where A, m, n, R₁, R₂, R₃, R₄ and R₅ in the reaction formula are as defined above.

In some embodiments, the base is selected from the group consisting of sodium hydroxide, potassium carbonate, cesium carbonate, or sodium phosphate.

The compound of formula M7 and the compound of formula M8 are commercially available or can be chemically synthesized.

In some embodiments, the compound of formula M7 can be prepared by a method comprising: 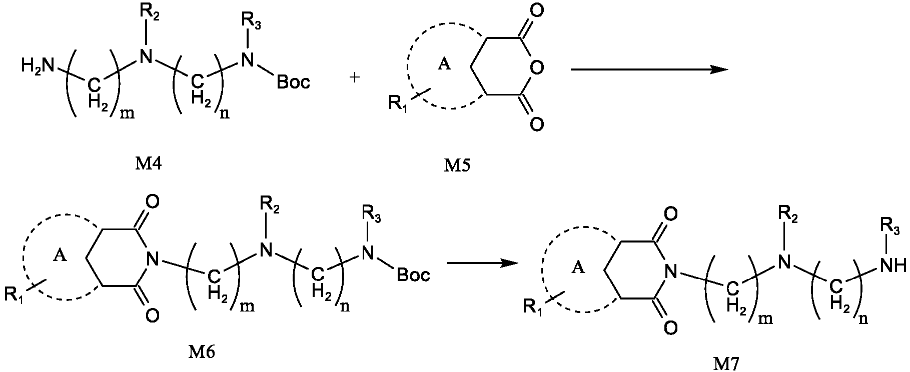 according to the above reaction formula, reacting a compound of formula M4 with a compound of formula M5 to generate a compound of formula M6, and treating the compound of formula M6 in the presence of an acid to obtain the compound of formula M7; where A, m, n, R₁, R₂ and R₃ in the reaction formula are as defined above.

In some embodiments, the acid may be a common acid, such as hydrochloric acid.

The compound of formula M4 is commercially available or can be chemically synthesized.

In some embodiments, the compound of formula M4 can be prepared by a method comprising: 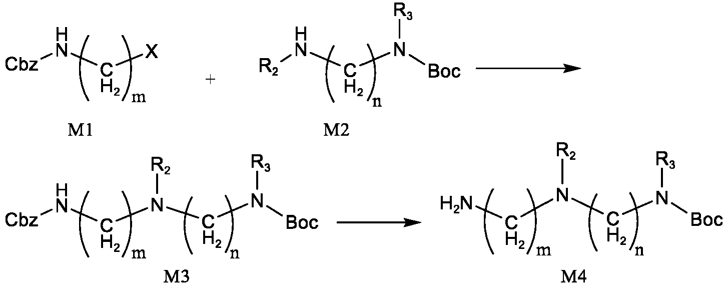 according to the above reaction formula, reacting a compound of formula M1 with a compound of formula M2 to generate a compound of formula M3, and hydrogenating the compound of formula M3 to obtain the compound of formula M4; where in the reaction formula, X is halogen, Cbz is benzyloxycarbonyl and Boc is tert-butoxycarbonyl, and m, n, R₂ and R₃ are as defined above.

In a third aspect, the present disclosure provides a use, that is, the use of the compound of formula I as described above in the preparation of a drug for treating a cell proliferative disease.

In some embodiments, the cell proliferative disease is cancer.

In some embodiments, the cancer is selected from the group consisting of digestive system tumor, respiratory system tumor and hematopoietic tumor, such as colon tumor, lung tumor, hematologic tumor, *etc.*

In a fourth aspect, the present disclosure provides a method for treating a cell proliferative disease, comprising administering a therapeutically effective amount of the compound of formula I as described above to a subject in need thereof.

In some embodiments, the cell proliferative disease is cancer.

In some embodiments, the cancer is selected from the group consisting of digestive system tumor, respiratory system tumor and hematopoietic tumor, such as colon tumor, lung tumor, hematologic tumor, *etc.*

The diimide derivative provided by the present disclosure is a novel compound, which has been found to have an excellent antitumor activity *in vivo* and *in vitro,* and have significant inhibitory effects on various tumors such as colon tumor, lung tumor, and hematologic tumor. Moreover, the antitumor activity of the diimide derivative is significantly better than that of similar compounds, thus having broad application prospects.

### DETAILED DESCRIPTION

The present disclosure is further described below with examples, and some preferred compounds will be exemplified. It should be noted that the examples are not intended to limit the compounds and technical effects of the present disclosure.

### Example 1: Compound I-01

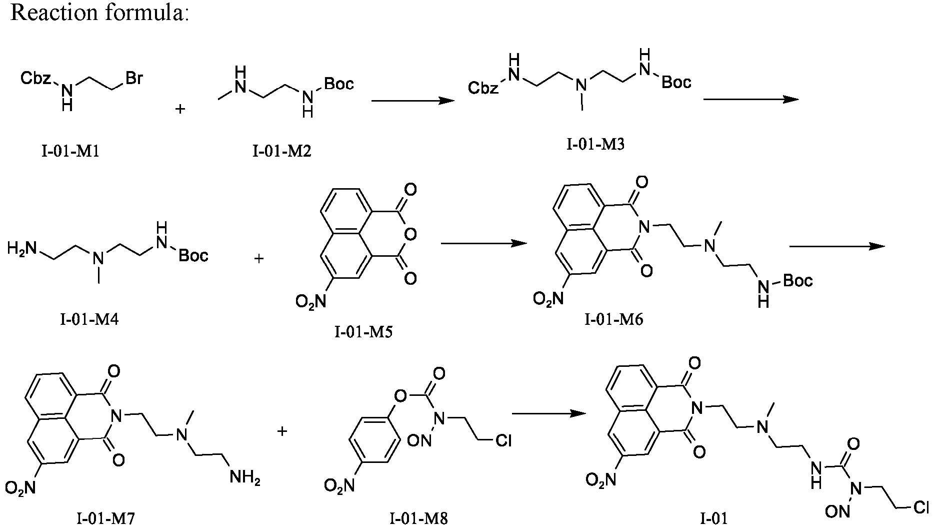

According to the reaction formula, the compound I-01 was prepared as follows:
1.1 Preparation of intermediate I-01-M3: compound I-01-M2 (3.48 g, 20 mmol) was dissolved in 30 mL of dimethyl formamide (DMF), and then K₂CO₃ (4.16 g, 30 mmol), NaI (3.00 g, 20 mmol), and compound I-01-M1 (5.14 g, 20 mmol) were successively added into a resulting mixed solution to allow reaction at 30 °C overnight. After the reaction was completed, the reaction solution was added with purified water (120 mL), and extracted with ethyl acetate (180 mL) for three times, dried with anhydrous MgSO₄ and filtered. The mother liquor was concentrated and purified by column chromatography to obtain the intermediate I-01-M3 (4.07 g, 11.6 mmol), with a yield of 58 %.
1.2 Preparation of intermediate I-01-M4: the intermediate I-01-M3 (4.07 g, 11.6 mmol) was dissolved in methanol (25 mL), and then added with 10 % Pd/C (0.3 g). The resulting solution was subjected to hydrogen replacement for three times, then reacted overnight under normal pressure, and filtered with diatomite. The mother liquor was concentrated to obtain colorless oily intermediate I-01-M4 (2.50 g, 11.5 mmol), with a yield of 99.1 %.
1.3 Preparation of intermediate I-01-M6: the intermediate I-01-M4 (2.50 g, 11.5 mmol), ethanol (60 mL) and compound I-01-M5 (2.79 g, 11.5 mmol) were successively added into a reaction flask, the resulting mixture was heated to 80 °C for reaction for 2 h, cooled, then concentrated and purified by column chromatography to obtain the intermediate I-01-M6 (3.63 g, 8.2 mmol), with a yield of 71.3 %.
1.4 Preparation of intermediate I-01-M7: the intermediate I-01-M6 (3.63 g, 8.2 mmol) was dissolved in a 4 % hydrogen chloride ethyl acetate solution (50 mL) to allow reaction overnight at room temperature, filtered, and dried to obtain the intermediate I-01-M7 (2.67 g, 7.8 mmol), with a yield of 95.1 %.
1.5 Synthesis of compound I-01: compound I-01-M8 (2.46 g, 9 mmol), dichloromethane (30 mL), and triethylamine (2.02 g, 20 mmol) were successively added into a reaction flask and cooled to 0 °C, the intermediate I-01-M7 (2.67 g, 7.8 mmol) was added portion-wise to allow reaction overnight at 0 °C. The reaction product was washed with water (20 mL), dried with anhydrous MgSO₄ and filtered. The mother liquor was concentrated and purified by column chromatography to obtain the compound I-01 (2.14 g, 4.5 mmol), with a yield of 57.7 %. Product identification: ¹H-NMR (400MHz, d₆-DMSO) δ: 2.97 (s, 3H), 3.34-3.40 (m, 2H), 3.46-3.51 (m, 2H), 3.58 (t, *J =* 6.5 Hz, 2H), 3.72-3.77 (m, 2H), 3.98-4.11 (m, 2H), 4.46 (t, *J =* 6.2 Hz, 2H), 8.06 (dd, *J =* 8.2, 7.4 Hz, 1H), 8.67-8.62 (dd, *J =* 7.3, 1.1 Hz, 1H), 8.78 (dd, *J =* 8.5, 1.1 Hz, 1H), 8.89 (d, *J =* 2.3 Hz, 1H), 9.00 (t, *J =* 5.5 Hz, 1H), 9.48 (d, *J =* 2.3 Hz, 1H).

### Example 2: Compound I-02

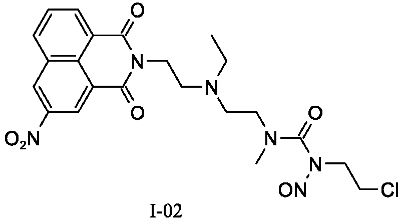

Compound I-02 was prepared with reference to the method of Example 1. Product identification: ¹H-NMR (400MHz, d₆-DMSO) δ: 1.04 (t, *J =* 7.6 Hz, 3H), 2.81-2.84 (m, 4H), 2.92 (s, 3H), 3.45-3.50 (m, 2H), 3.52-3.55 (m, 2H), 3.70 (t, *J =* 2.9 Hz, 2H), 3.82 (t, *J =* 2.9 Hz, 2H), 4.46 (t, *J =* 4.6 Hz, 2H), 8.06 (dd, *J=* 8.2, 7.4 Hz, 1H), 8.67-8.62 (dd, *J =* 7.3, 1.1 Hz, 1H), 8.78 (dd, *J=* 8.5, 1.1 Hz, 1H), 8.89 (d, *J* = 2.3 Hz, 1H), 9.00 (t, *J* = 5.5 Hz, 1H), 9.48 (d, *J =* 2.3 Hz, 1H).

### Example 3: Compound I-03

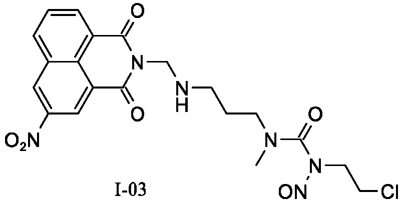

Compound I-03 was prepared with reference to the method of Example 1. Product identification: ¹H-NMR (400MHz, d₆-DMSO) δ: 1.73-1.79 (m, 2H), 2.94 (s, 3H), 3.25 (t, *J =* 5.1 Hz, 2H), 2.47-2.53 (m, 2H), 3.55 (t, *J* = 2.9 Hz, 2H), 3.78 (t, *J =* 2.9 Hz, 2H), 4.76 (d, *J =* 2.9 Hz, 2H), 8.06 (dd, *J =* 8.2, 7.4 Hz, 1H), 8.67-8.62 (dd, *J =* 7.3, 1.1 Hz, 1H), 8.78 (dd, *J =* 8.5, 1.1 Hz, 1H), 8.90 (d, *J =* 2.3 Hz, 1H), 9.00 (t, *J =* 5.5 Hz, 1H), 9.48 (d, *J=* 2.3 Hz, 1H).

### Example 4: Compound I-04

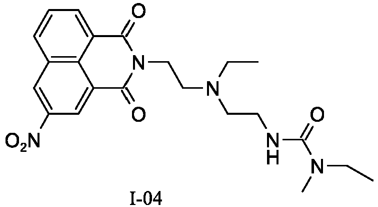

Compound I-04 was prepared with reference to the method of Example 1. Product identification: ¹H-NMR (400MHz, d₆-DMSO) δ: 1.04 (t, *J* =7.6 Hz, 3H), 1.18 (t, J =7.6Hz, 3H), 2.64-2.68 (m, 4H), 2.96(s, 3H), 3.28 (q, *J =* 5.8 Hz, 2H), 3.44 (t, *J =* 4.5Hz 2H), 3.78-3.82 (m, 2H), 4.44 (t, *J =* 4.4 Hz 2H), 8.06 (dd, *J =* 8.2, 7.4 Hz, 1H), 8.67-8.62 (dd, *J =* 7.3, 1.1 Hz, 1H), 8.81 (dd, *J =* 8.5, 1.1 Hz, 1H), 8.89 (d, *J* = 2.3 Hz, 1H), 9.07 (t, *J* = 5.5 Hz, 1H), 9.48 (d, *J* = 2.3 Hz, 1H).

### Example 5: Compound I-05

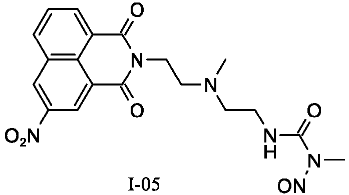

Compound I-05 was prepared with reference to the method of Example 1. Product identification: ¹H-NMR (400MHz, d₆-DMSO) δ: 2.98 (s, 3H), 3.16 (s,3H), 3.44-3.47 (m, 2H), 3.56-3.63 (m, 4H), 4.33 (t, *J =* 4.6 Hz, 2H), 8.06 (dd, *J =* 8.2, 7.4 Hz, 1H), 8.67-8.62 (dd, *J =* 7.3, 1.1 Hz, 1H), 8.81 (dd, *J =* 8.5, 1.1 Hz, 1H), 8.89 (d, *J =* 2.3 Hz, 1H), 9.02 (t, *J =* 5.5 Hz, 1H), 9.51 (d, *J =* 2.3 Hz, 1H).

### Example 6: Compound I-06

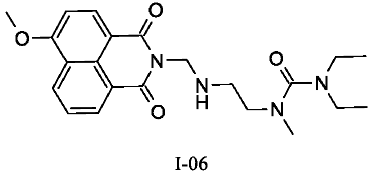

Compound I-06 was prepared with reference to the method of Example 1. Product identification: ¹H-NMR (400MHz, d₆-DMSO) δ: 1.17 (t, *J =* 5.8 Hz, 6H), 2.87 (s, 3H), 3.15 (q, *J =* 5.8 Hz, 4H), 3.22-3.25 (m, 2H), 3.38 (t, *J =* 4.5 Hz, 2H), 3.89 (s, 3H), 4.86(s, 2H), 7.65 (t, *J =* 7.8 Hz, 1H), 8.06 (dd, *J =* 8.2, 7.4 Hz, 1H), 8.13 (d, *J =* 8.4 Hz, 1H), 8.32-8.42 (m, 2H), 8.62-8.68 (m, 1H).

### Example 7: Compound I-07

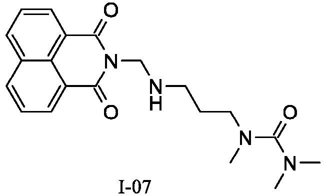

Compound I-07 was prepared with reference to the method of Example 1. Product identification: ¹H-NMR (400MHz, d₆-DMSO) δ: 1.77-1.83(m, 2H), 2.91 (s,3H), 2.94 (s, 6H),3.25 (t, *J =* 5.1 Hz, 2H), 3.54-3.58 (m, 2H), 4.84 (d, *J =* 3.5 Hz, 2H), 7.65 (d, *J =* 7.4 Hz, 1H), 7.83-7.86 (m, 2H), 8.43-8.52 (m, 4H).

### Example 8: Compound I-08

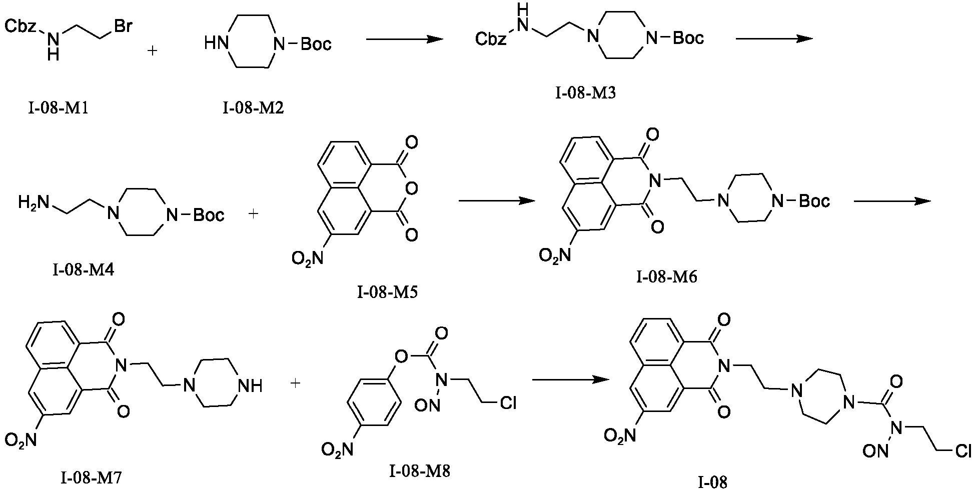

According to the reaction formula, compound I-08 was prepared by a specific method as follows:
8.1 Synthesis of intermediate I-08-M3: compound I-08-M2 (3.72 g, 20 mmol) was dissolved in 30 mL of DMF; and then K₂CO₃ (4.16 g, 30 mmol), NaI (3.00 g, 20 mmol), and compound I-08-M1 (5.14 g, 20 mmol) were successively added into a resulting mixed solution to allow reaction overnight at 30 °C. After the reaction was completed, the reaction solution was added with purified water (120 mL), extracted with ethyl acetate (180 mL) for three times, dried with anhydrous MgSO₄ and filtered. The mother liquor was concentrated and purified by column chromatography to obtain the intermediate I-08-M3 (4.39 g, 12.1 mmol), with a yield of 60.5 %.
8.2 Synthesis of intermediate I-08-M4: the intermediate I-08-M3 (4.39 g, 12.1 mmol) was dissolved in methanol (20 mL), and then added with 10% Pd/C (0.3 g). The resulting solution was subjected to hydrogen replacement for three times, reacted overnight under normal pressure, and filtered with diatomite. The mother liquor was concentrated to obtain the colorless oily intermediate I-08-M4 (2.75 g, 12.0 mmol), with a yield of 99.2 %.
8.3 Synthesis of intermediate I-08-M6: the intermediate I-08-M4 (2.75 g, 12.0 mmol), ethanol (50 mL), and compound I-08-M5 (2.92 g, 12.0 mmol) were successively added into a reaction flask, the resulting mixture was heated to 80 °C for reaction for 2 h. The reaction product was cooled, concentrated and purified by column chromatography to obtain the intermediate I-08-M6 (4.22 g, 9.3 mmol), with a yield of 77.5 %.
8.4 Synthesis of intermediate I-08-M7: the intermediate I-08-M6 (4.22 g, 9.3 mmol) was dissolved in a 4 % hydrogen chloride ethyl acetate solution (60 mL) to allow reaction overnight at room temperature. The reaction product was filtered, and dried to obtain the intermediate I-08-M7 (3.12 g, 8.8 mmol), with a yield of 94.6 %.
8.5 Synthesis of compound I-08: compound I-08-M8 (2.62 g, 9.6 mmol), dichloromethane (35 mL), and triethylamine (2.02 g, 20 mmol) were successively added into a reaction flask and cooled to 0 °C, intermediate I-08-M7 (3.12 g, 8.8 mmol) was added portion-wise to allow reaction overnight at 0°C. The reaction product was washed with water (25 mL), dried with anhydrous MgSO₄ and filtered. The mother liquor was concentrated and purified by column chromatography to obtain the compound I-08 (2.54 g, 5.2 mmol), with a yield of 59.1 %. Product identification: ¹H-NMR (400MHz, d₆-DMSO) δ: 2.61-2.73 (m, 4H), 2.94-2.98 (m, 2H), 3.19-3.34 (m, 4H), 3.57 (t, *J =* 2.9 Hz, 2H), 3.78 (t, *J =* 2.9 Hz, 2H), 4.45 (t, *J =* 4.6 Hz, 2H), 8.67-8.62 (dd, *J =* 7.3, 1.1 Hz, 1H), 8.78 (dd, *J =* 8.5, 1.1 Hz, 1H), 8.89 (d, *J =* 2.3 Hz, 1H), 9.00 (t, *J =* 5.5 Hz, 1H), 9.48 (d, *J* = 2.3 Hz, 1H).

### Example 9: Compound I-09

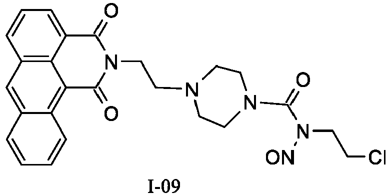

Compound I-09 was prepared with reference to the method of Example 1. Product identification: ¹H-NMR (400MHz, d₆-DMSO) δ: 2.61-2.73 (m, 4H), 2.94-2.98 (m, 2H), 3.19-3.34 (m, 4H), 3.57 (t, *J =* 2.9 Hz, 2H), 3.78 (t, *J =* 2.9 Hz, 2H), 4.47(t, *J =* 4.6 Hz, 2H), 7.53 (t, *J =* 6.1 Hz, 1H), 7.58-7.65 (m, 1H), 7.79 (t, *J =* 7.8 Hz, 1H), 8.04-8.10 (m, 1H), 8.33-8.35 (m, 1H), 8.49 (d, *J =* 6.3Hz, 1H), 8.84 (s, 1H), 9.38(d, *J* = 7.8 Hz, 1H).

### Example 10: Compound I-10

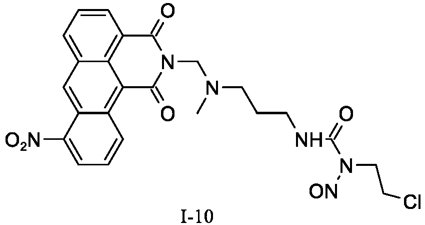

Compound I-10 was prepared with reference to the method of Example 1. Product identification: ¹H-NMR (400MHz, d₆-DMSO) δ: 1.72-177 (m, 2H), 2.95 (s, 3H), 3.35-3.38 (m, 2H), 3.54 (t, *J =* 2.9 Hz, 2H), 3.68 (t, *J =* 5.1 Hz, 2H),3.82 (t, *J =* 2.9 Hz, 2H),4.65 (s, 2H), 7.73-7.81 (m, 2H),8.27 (d, *J =* 6.2 Hz, 1H), 8.39-8.50 (m, 2H), 8.87 (d, *J =* 6.2 Hz, 1H), 9.28 (d, *J* = 2.6 Hz, 1H), 9.52 (d, *J =* 7.9 Hz, 1H).

### Example 11: Compound I-11

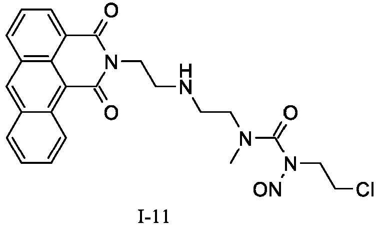

Compound I-11 was prepared with reference to the method of Example 1. Product identification: ¹H-NMR (400MHz, d₆-DMSO) δ:2.94 (s, 3H), 2.95-3.05 (m, 2H), 3.40 (t, *J* = 4.5 Hz, 2H), 3.56 (t, *J =* 2.9 Hz, 2H), 3.80-3.82 (m, 2H), 3.84 (t, *J =* 2.9 Hz, 2H), 4.49 (t, *J =* 4.5 Hz, 2H), 7.53 (t, *J* = 6.1 Hz, 1H),7.59-7.66 (m, 1H),7.78 (t, *J =* 6.2 Hz, 1H), 8.04-8.10 (m, 2H), 8.33-8.35 (m, 1H), 8.49 (d, *J=* 6.2 Hz, 1H),8.73 (s, 1H), 9.38(d, *J =* 7.8 Hz, 1H).

### Example 12: Compound 1-12

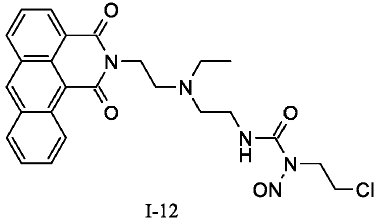

Compound I-12 was prepared with reference to the method of Example 1. Product identification: ¹H-NMR (400MHz, d₆-DMSO) δ: 1.04 (t, *J =* 7.2 Hz, 3H), 2.56 (q, *J* = 5.8 Hz, 2H), 2.94 (t, *J =* 4.4 Hz, 2H), 3.27-3.31 (m, 2H), 3.45 (t, *J=* 4.4 Hz, 2H), 3.54 (t, *J =* 2.9 Hz, 2H), 3.77 (t, *J* = 2.9 Hz, 2H), 4.14 (t, *J* = 4.6 Hz, 2H),7.53 (t, *J =* 6.2 Hz, 1H),7.59-7.66 (m, 1H),7.78 (t, *J =* 6.2 Hz, 1H), 8.04-8.10 (m, 2H), 8.33-8.35 (m, 1H), 8.49 (d, *J =* 6.2 Hz, 1H), 8.73 (s, 1H), 9.38 (d, *J =* 7.8 Hz, 1H).

### Example 13: Study on the in vitro antitumor activity of the compound of formula I

Objective: to test the antiproliferative activity of the compound in the present disclosure against a variety of tumor cells.

Test compounds: compounds 1-01, I-02, I-03, I-04, I-05, I-06, I-07, I-08, I-09, I-10, I-11, I-12 of the present disclosure; positive drugs: amonafide and lomustine; and comparative compounds: 1, 1d, Cob280, and Cob281. The compound 1 refers to "compound 1" described in "Journal of Experimental Therapeutics and Oncology, 2005, 5: p 15-22", the compound 1d refers to "1d" described in "Journal of Cancer Molecules, 2010, 5(2): p 41-47", the compound Cob280 refers to "Cob280" described in Example 17a of patent WO2012104788, the compound Cob281 refers to "Cob281" in Example 17b of patent WO2012104788, and they were all prepared according to the method in the literature. Amonafide and lomustine were commercially available.

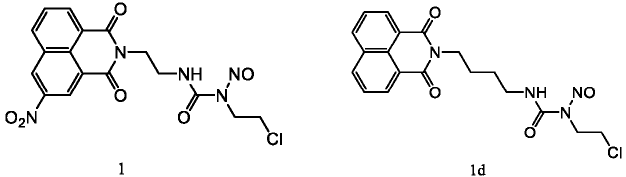

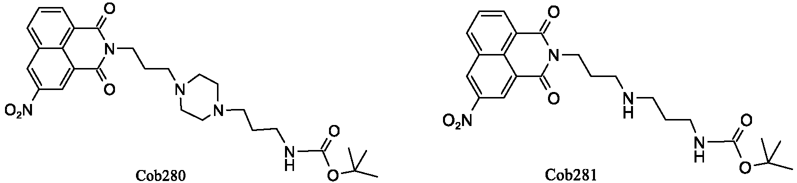

Test cells: human colon cancer cells (HT-29, COLO 205), human lung cancer cells (NCI-H460, A549), and human leukemia cells (HL-60, U-937).

Test methods: each tumor cell was processed separately. Cells were cultured in a culture medium at 37 °C for 24 h for later use. The cells in the exponential growth phase were harvested, treated with trypsin to prepare a cell suspension, the cell suspension was centrifuged, and then the cell pellet was resuspended in a small amount of fresh culture medium as a stock cell solution. This stock solution was diluted to the desired cell concentration, and the concentration of each cell was as shown in Table 1:

**Table 1: concentrations of each tumor cell**

| Cell line | Cell concentration (cell count/mL) |
|---|---|
| HT-29 | 30000 |
| COLO 205 | 15000 |
| MCI-H460 | 15000 |
| A549 | 20000 |
| HL-60 | 30000 |
| U-937 | 30000 |

A 96-well plate was taken, and blank control groups, cell control groups and compound treatment groups were set up. In the blank control groups, only 10 % phosphate buffer saline (PBS) was added to each well. 100 µL of cells at the above concentration were added into each well of the cell control groups and compound treatment groups. The remaining wells were filled with 200 µL of 10 % PBS. The plate was placed in an incubator overnight. 100 µL of each test compound at different dilution concentrations was added into each well of the compound treatment groups, and the dilution concentrations of the compound were as shown in Table 2.

**Table 2: dilution concentration of test compound**

| No. | Compound concentration (µM) |
|---|---|
| 1 | 200 |
| 2 | 66.7 |
| 3 | 22.2 |
| 4 | 7.41 |
| 5 | 2.47 |
| 6 | 0.823 |
| 7 | 0.274 |
| 8 | 0.0914 |

After adding the drug, the plate was placed in an incubator to be cultured for 96 hours, 22 µL of resazurin sodium solution (Alarm blue, SIGMA R7017) was added into each well, and the plate was returned to the incubator to be cultured for another 4 h. The plate was shaken for 10 s after being taken out. A fluorescence value of each well was tested at 530/590nm.

The above operations were repeated for three times.

The IC₅₀ value of each test compound was calculated with the Prism7 software. The IC₅₀ was divided into different grades according to value, that is: SSS:<0.5µM, SS:0.5-1µM; S:1-5µM; A:5-10µM; B:11-20µM; C:21-50µM; D:51-100µM; E:>100µM. The main results are as shown in Table 3:

**Table 3: IC₅₀ grades of compound's inhibitory effect on tumor cells**

| Compound | HT-29 | COLO 205 | NCI-H460 | A549 | HL-60 | U-937 |
|---|---|---|---|---|---|---|
| I-01 | SSS | SSS | SSS | SS | SSS | SSS |
| I-02 | SS | S | S | SS | SS | S |
| I-03 | S | S | S | S | S | SS |
| I-04 | S | A | S | S | S | S |
| I-05 | SS | S | S | SS | SS | SS |
| I-06 | S | A | A | S | S | S |
| I-07 | A | S | S | A | S | A |
| I-08 | S | SS | S | SS | S | SS |
| I-09 | S | S | S | S | S | SS |
| I-10 | SS | SS | S | S | SS | SS |
| I-11 | S | S | A | S | A | S |
| I-12 | S | SS | S | S | SS | S |
| 1 | C | C | B | A | B | C |
| 1d | C | C | D | C | C | B |
| Cob280 | A | B | B | B | A | A |
| Cob281 | A | B | B | A | A | A |
| Amonafide | B | A | B | S | A | B |
| Lomustine | D | E | D | E | C | B |

Test results show that lomustine has a poor activity against various tumor cells, while amonafide has a better activity. The activities of compounds 1d, 1, Cob280 and Cob281 are not better than that of amonafide, and even is reduced. But surprisingly, the antitumor activity of the compound of the present disclosure is significantly better than that of amonafide, lomustine, and similar compounds 1d, 1, Cob280, and Cob281. The activities of some preferred compounds of the present disclosure are more than 100 times or above than those of similar compounds.

### Example 22: Study on the in vivo anti-tumor effect of the compounds of formula I

Objective: to further test the *in vivo* antitumor effects of compounds of the present disclosure Test compounds: I-01, I-08.

Test animals: 5-week-old female BALB/c nude mice were fed normally for 7 days, and mice with good physical conditions were selected for experiments.

Tumor cells: human colon cancer cells COLO 205 were subjected to cell culture in a RPMI-1640 medium containing 10 % fetal bovine serum. The passaging was carried out for 1-2 times a week, with a passaging ratio of 1:3 to 1:6.

Modeling and grouping: COLO 205 cells in a logarithmic growth phase were harvested and injected into the anterior right mammary fat pad of nude mice with an injection volume of 5×10⁶ cells per animal to establish a COLO 205 transplanted tumor model. The animal status was observed regularly, tumor diameter was measured, and tumor volume was calculated. After the tumor volume reached approximately 150 mm³, tumor-bearing mice in good health and with similar tumor volumes were selected and randomly divided into a solvent control group, an I-01 group, and an I-08 group.

Administration: each test compound was dissolved in solvent, and each animal group was treated with corresponding compounds or blank solvent, respectively. The administration method was intragastric administration, with a dosage of 10 mg/kg, once a day, and a total of 21 times.

Result: on the last day of the experiment, tumor diameter and tumor weight were measured, tumor growth inhibition rate (GI) and tumor weight inhibition rate (IR) were calculated. The main results are as shown in Table 4.

Tumor growth inhibition rate=100%×[1-(Tvt-Tvini)÷(Cvt-Cvini)], where Tvt represents tumor volume of the treatment group at the end of the experiment, Tvini represents initial tumor volume of the treatment group, Cvt represents tumor volume of the solvent control group at the end of the experiment, and Cvini represents initial tumor volume of the solvent control group.

Tumor weight inhibition rate=100%×(Cw-Tw)÷Cw, where Cw represents tumor weight of the solvent control group at the end of the experiment, and Tw represents tumor weight of the treatment group at the end of the experiment.

**Table 4: in vivo tumor inhibitory effects of compounds**

| Compound | Tumor growth inhibition rate (%) | Tumor weight inhibition rate (%) |
|---|---|---|
| I-01 | 75.3% | 69.3% |
| I-08 | 43.5% | 31.2% |

The test results show that the compound of the present disclosure also has a strong antitumor effect in vivo.

Although the foregoing has been described the present disclosure in detail with the general description and specific embodiments, some modifications or improvements can be made by those skilled in the art on the basis of the present disclosure, and these modifications or improvements made without departing from the spirit of the present disclosure all fall within the scope of protection of the present disclosure as claimed.

## Claims

1. A diimide derivative represented by formula I, 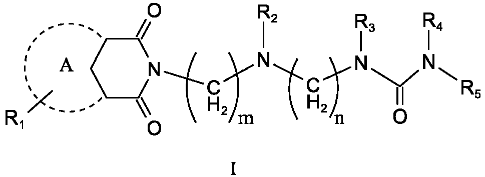
wherein in formula I,
A is naphthalene or anthracene, fused with diimide through 3 carbon atoms;
Ri is hydrogen, alkyloxy or nitro;
m or n is 1, 2 or 3, and m+n ≤ 4;
R₂ is alkyl;
R₃ is hydrogen or alkyl; and
R₄ or R₅ is alkyl, haloalkyl or nitroso.

2. The diimide derivative according to claim 1, wherein the alkyl is C1-C4 alkyl, the alkyloxy is C1-C4 alkyloxy, and the haloalkyl is C1-C4 haloalkyl.

3. The diimide derivative according to claim 1, wherein A is naphthalene.

4. The diimide derivative according to claim 1, wherein Ri is nitro or C1-C4 alkyloxy.

5. The diimide derivative according to claim 1, wherein R₃ is hydrogen.

6. The diimide derivative according to claim 1, wherein R₂ forms a ring together with R₃.

7. The diimide derivative according to claim 1, wherein R₄ is C1-C4 haloalkyl, and R₅ is nitroso.

8. The diimide derivative according to claim 1, wherein m and n are: m=1 and n=3, m=1 and n=2, or m=2 and n=2.

9. A method for preparing a diimide derivative represented by formula I, comprising: 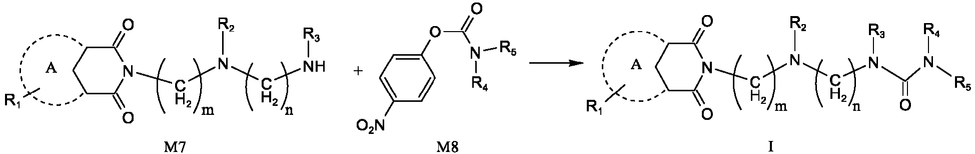 reacting a compound of formula M7 with a compound of formula M8 in the presence of a base to generate a compound of formula I according to the above reaction formula; wherein A, m, n, R₁, R₂, R₃, R₄ and R₅ in the reaction formula are as defined in claim 1.

10. The method according to claim 9, wherein the compound of formula M7 is prepared by a method comprising: 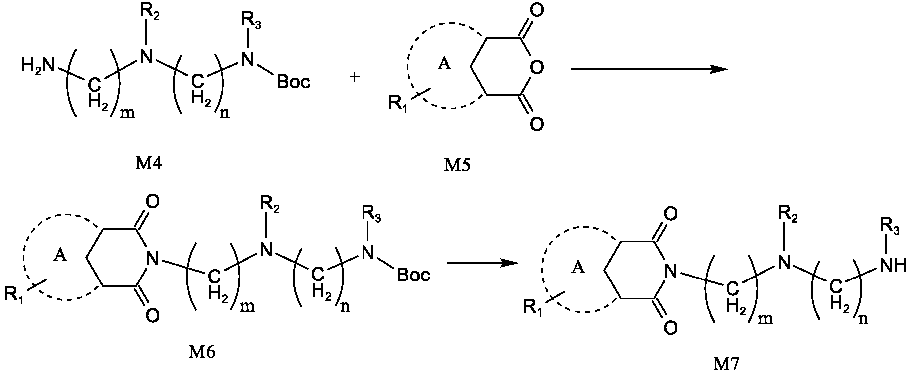 according to the above reaction formula, reacting a compound of formula M4 with a compound of formula M5 to generate a compound of formula M6, and treating the compound of formula M6 in the presence of an acid to obtain the compound of formula M7; wherein A, m, n, R₁, R₂ and R₃ in the reaction formula are as defined in claim 1.

11. The method according to claim 10, wherein the compound of formula M4 is prepared by a method comprising: 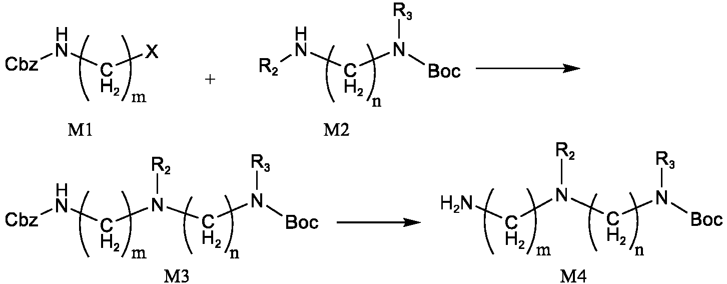 according to the above reaction formula, reacting a compound of formula M1 with a compound of formula M2 to generate a compound of formula M3, and hydrogenating the compound of formula M3 to obtain the compound of formula M4; wherein in the above reaction formula, X is halogen, Cbz is benzyloxycarbonyl and Boc is tert-butoxycarbonyl, m, n, R₂ and R₃ are as defined in claim 1.

12. Use of the diimide derivative according to any one of claims 1 to 8 in the preparation of a drug for treating a cell proliferative disease.

13. The use according to claim 12, wherein the cell proliferative disease is cancer.
